# EUROPEAN PATENT SPECIFICATION

(11) **EP 4 561 671 B1**
(45) Date of publication and mention of the grant of the patent: **04.11.2026**
(21) Application number: 23757741.6
(22) Date of filing: 25.07.2023
(51) Int. Cl.: A61N 1/05, A61N 1/36, A61N 1/365

(54) **IMPLANTABLE MEDICAL DEVICE HAVING AN INERTIAL SENSING UNIT**
IMPLANTIERBARE MEDIZINISCHE VORRICHTUNG MIT TRÄGHEITSMESSEINHEIT
DISPOSITIF MÉDICAL IMPLANTABLE AYANT UNE UNITÉ DE DÉTECTION INERTIELLE

(30) Priority: 26.07.2022 US 202263392291 P
(43) Date of publication of application: 04.06.2025
(73) Proprietor: The Alfred E. Mann Foundation for Scientific Research, Valencia, CA 91355 (US)
(72) Inventor: SHELTON, Brian, Ventura, California 93004 (US); MECH, Brian, Buffalo, Minnesota 55313 (US); ELYAHOODAYAN, Sahar, Los Angeles, California 90066 (US)
(74) Representative: Mewburn Ellis LLP
(86) International application number: PCT/US2023/028576
(87) International publication number: WO 2024/025880

(56) References cited:
- EP-A1- 3 184 045
- US-A1- 2015 157 864
- US-A1- 2016 121 110
- US-A1- 2018 333 578
- US-A1- 2021 379 381

## Description

### BACKGROUND

### 1. Field

The present disclosure relates to implantable medical devices configured to determine physiological data of a patient with an inertial sensing unit.

### 2. Description of the Related Art

Implantable pulse generators (IPGs) are used for a variety of therapeutic treatments in a patient, such as neurostimulation, cardiac stimulation, and/or spinal cord stimulation. An implanted accelerometer and/or gyroscope in the IPG may be used to detect a patient's heart rate and respiration rate. The intensity of these detected signals may vary not only per patient but also per time of day. This is especially so for respiration detection wherein the intensity of the respiration signal will drop when a patient sleeps. Thus, it may be required to set the accelerometer and gyro to their most sensitive settings. Patient motion often results in a much stronger signal that will not only swamp the respiration or heart-rate signals but can also cause these sensors to hit their rails (i.e., maximum and minimum values). When the sensor output hits the rails, the output is non-linear and may be difficult if not impossible to filter out from the much lower respiration and heart rate signals that generally have different frequency components.

EP3184045A1 discloses a system and a method for automatically implementing a therapy for treating sleep disordered breathing.

### SUMMARY

The invention is defined in the appended claims. Aspects, embodiments and examples disclosed herein which do not fall within the scope of the appended claims do not form part of the invention, and are merely provided for illustrative purposes.

The present disclosure relates to various embodiments of a stimulation system configured to be implanted in a patient. In one embodiment, the stimulation system includes an implantable pulse generator (IPG) and an implantable lead system. The IPG includes a processor, a memory device, a power supply, and an inertial sensing unit configured to measure physiological data such as one or more physiological indicators of the patient. The implantable lead system includes at least one electrical lead coupled to the power supply and at least one electrode at a distal end of the electrical lead. The memory device includes instructions that cause the IPG to receive the physiological indicators within an initial range; increase the initial range to an enlarged range in response to the physiological indicators crossing a threshold maximum value or a threshold minimum value of the initial range; receive the physiological indicators within the enlarged range, analyze the physiological indicators, and deliver stimulation to the patient through the electrode in response to the physiological indicators.

The present disclosure also relates to various embodiments of a method of providing stimulation to a patient for treatment of one or more medical conditions. In one embodiment, the method includes measuring, with an inertial sensing unit of an implantable pulse generator implanted in the patient, one or more physiological indicators of the patient within an initial range; increasing the initial range to an enlarged range in response to at least one of the physiological indicators crossing a threshold maximum value or a threshold minimum value of the initial range; receiving the one or more physiological indicators within the enlarged range; analyzing the one or more physiological indicators; and delivering stimulation to the patient, through at least one implantable lead coupled to the implantable pulse generator, in response to the one or more physiological indicators.

This summary is provided to introduce a selection of features and concepts of embodiments of the present disclosure that are further described below in the detailed description. This summary is not intended to identify key or essential features of the claimed subject matter, nor is it intended to be used in limiting the scope of the claimed subject matter. One or more of the described features or tasks may be combined with one or more other described features or tasks to provide a workable device or method.

### BRIEF DESCRIPTION OF THE DRAWINGS

The features and advantages of embodiments of the present disclosure will become more apparent by reference to the following detailed description when considered in conjunction with the following drawings. In the drawings, like reference numerals are used throughout the figures to reference like features and components. The figures are not necessarily drawn to scale.
FIGS. 1A-1B are a perspective view and a schematic view, respectively, of an implantable pulse generator (IPG) according to one embodiment of the present disclosure;
FIGS. 2A-2C are graphs depicting a physiological signal within an initial range/scale, the physiological signal clipping the initial range/scale, and the physiological signal within an enlarged range, respectively, according to one embodiment of the present disclosure; and
FIG. 3 is a flowchart is a method of operating an implantable medical device implanted in a patient according to one embodiment of the present disclosure.

### DETAILED DESCRIPTION

The present disclosure relates to various embodiments of an implantable device configured to measure or determine one or more physiological indicators that may include physiological signals of an individual, such as heart rate and/or respiration rate. In one or more embodiments, the implantable device measures or determines the physiological signal(s) with an inertial sensing unit that includes one or more inertial sensors, such as a gyroscope and/or an accelerometer (e.g., an inertial measurement unit (IMU) including a gyroscope and an accelerometer), which may be less invasive than the electrocardiogram (EKG) method that utilizes implanted electrodes.

The implantable device is configured to receive the physiological signal(s) from the inertial sensor within a range of values (e.g., the acceleration measured by the accelerometer between a maximum acceleration and a minimum acceleration). The range of the inertial sensor may be minimized to increase the resolution and thus the sensitivity of the inertial sensor, which may enable detection of low-intensity physiological signals. The implantable device is also configured to dynamically increase the range (e.g., increase the minimum and maximum acceleration of the range) in response to the physiological signal reaching the maximum or the minimum (or reaching a threshold near the maximum or minimum values), which may be caused by bulk movements of the patient, such as movements during exercise or body roll while sleeping. In this manner, the implantable device is configured to prevent clipping of the physiological signal, which would otherwise introduce non-linearities and broad-spectrum noise that would prevent or inhibit analysis of the physiological signals of interest.

The implantable device is also configured to filter or separate out the physiological signal from signals that result from bulk patient motions, such as with a bandpass filter, and to stimulate and/or adjust the stimulation delivered by the implantable device to a nerve or a muscle of the patient in response to the physiological signal(s).

The terminology utilized herein is for the purpose of describing particular embodiments only and is not intended to be limiting of the present disclosure. As utilized herein, the singular forms "a", "an" and "the" are intended to include the plural forms as well, unless the context clearly indicates otherwise. As utilized herein, the term "and/or" includes any and all combinations of one or more of the associated listed items.

It will be understood that, although the terms "first", "second", "third", etc., may be utilized herein to describe one or more suitable elements, components, regions, and/or sections, these elements, components, regions, and/or sections should not be limited by these terms. These terms are only utilized to distinguish one element, component, region, layer or section from another element, component, region, layer or section. Thus, a first element, component, region, or section discussed could be termed a second element, component, region, or section, without departing from the spirit and scope of the present disclosure.

It will be understood that when an element is referred to as being "on", "connected to", "coupled to", or "adjacent to" another element, it can be directly on, connected to, coupled to, or adjacent to the other element, or one or more intervening element(s) may be present. In contrast, when an element is referred to as being "directly on," "directly connected to", "directly coupled to", or "immediately adjacent to" another element, there are no intervening elements present.

As utilized herein, the term "substantially" and similar terms are utilized as terms of approximation and not as terms of degree, and are intended to account for the inherent deviations in measured or calculated values that would be recognized by those of ordinary skill in the art. Also, the terms "about," "approximately," and similar terms, when utilized herein in connection with a numerical value or a numerical range, are inclusive of the stated value and refer to within an acceptable range of deviation for the particular value as determined by one of ordinary skill in the art, considering the measurement in question and the error associated with measurement of the particular quantity (e.g., the limitations of the measurement system).

Also, any numerical range recited herein is intended to include all subranges of the same numerical precision subsumed within the recited range. For example, a range of "1.0 to 10.0" is intended to include all subranges between (and including) the recited minimum value of 1.0 and the recited maximum value of 10.0, that is, having a minimum value equal to or greater than 1.0 and a maximum value equal to or less than 10.0, such as, for example, 2.4 to 7.6. Any maximum numerical limitation recited herein is intended to include all lower numerical limitations subsumed therein and any minimum numerical limitation recited in this specification is intended to include all higher numerical limitations subsumed therein. Accordingly, Applicant reserves the right to amend this specification, including the claims, to expressly recite any sub-range subsumed within the ranges expressly recited herein.

Example embodiments of the present disclosure will now be described with reference to the accompanying drawings. In the drawings, the same or similar reference numerals refer to the same or similar elements throughout. As utilized herein, the utilize of the term "may," when describing embodiments of the present disclosure, refers to "one or more embodiments of the present disclosure."

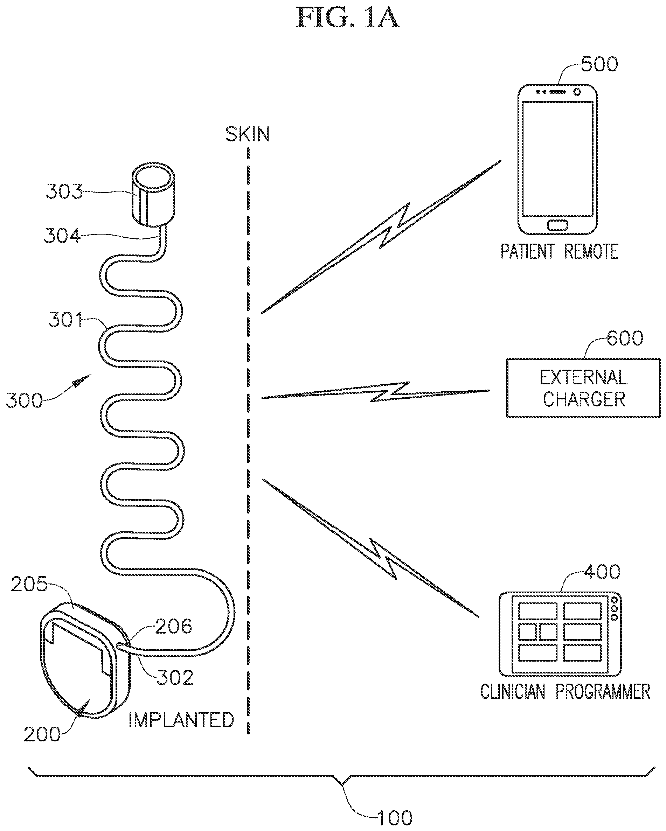

FIGS. 1A-1B are a perspective view and a schematic block diagram, respectively, of a stimulation system **100** configured to treat a patient via electrical stimulation, such as neurostimulation, cardiac stimulation, or spinal cord stimulation according to one embodiment of the present disclosure. The stimulation system **100** may include an implantable pulse generator (IPG) 200, and an implantable lead system **300** coupled to the IPG **200,** implanted in a patient. In the illustrated embodiment, the implantable lead system **300** includes at least one electrical lead **301** having a proximal end **302** coupled to the IPG **200,** and an electrode **303** coupled to a distal end **304** of the electrical lead **301** to periodically deliver an electric current pulse for a variety of therapeutic treatments for the patient, such as neurostimulation, cardiac stimulation, and/or spinal cord stimulation. The type or kind of the electrode **303** may be selected based on the location and the type of nerves stimulated (e.g., a cuff electrode to stimulate a nerve bundle, such as the hypoglossal nerve or the vagus nerve; a cardiac electrode to stimulate heart/myocardium; or a spinal cord lead, such as a paddle or a linear lead, to stimulate the spinal cord). The IPG **200** and the implantable lead system **300** may be implanted in any suitable locations in the patient depending on the therapeutic treatment delivered by the system 100. For example, the IPG **200** may be implanted in a subcutaneous pocket in the upper chest of the patient, and the electrical lead(s) **301** may extend from the IPG **200** through the superior vena cava such that the electrode(s) **303** are connected to the myocardium of the patient's heart. In one or more embodiments, the IPG **200** may be an obstructive sleep apnea (OSA) stimulator device implanted in a subcutaneous pocket in the upper chest of the patient, and the electrical lead(s) **301** may extend from the IPG **200** such that the electrode(s) **303** are connected to one or more of the patient's upper airway dilator muscles.

In the illustrated embodiment, the stimulation system **100** also includes a clinician programmer (CP) device **400** and a patient remote (PR) device **500** each electronically coupled to (i.e., in wireless RF communication with) the IPG **200,** and an external charger **600** configured to charge the IPG **200** via inductive coupling.

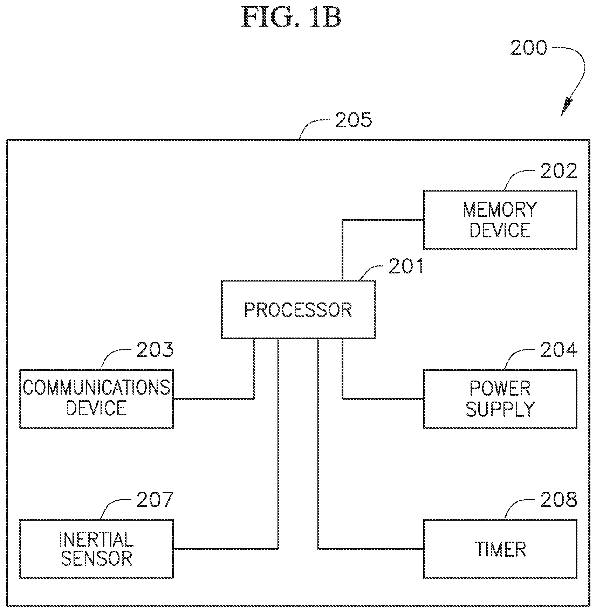

With reference now to the embodiment illustrated in FIG. 1B, the IPG **200** includes a processor (e.g., a processing circuit) **201,** a non-volatile memory device **202** (e.g., flash memory, or read-only memory (ROM), such as programmable read-only memory (PROM) or erasable programmable read-only memory (EPROM)), a communications device **203** (e.g., a receiver and a transmitter, or a transceiver), and a power supply **204** (e.g., a primary battery or an inductively chargeable rechargeable battery). The communications device **203** provides wireless communication links through the skin of the patient to the CP device **400** and the PR device 500. Wireless links may include Bluetooth ^{™}, Bluetooth Low Energy or other protocols with suitable authentication and encryption to protect patient data. The processor **201,** the non-volatile memory device **202,** the communications device **203,** and the power supply **204** are in communication with each. Additionally, in the illustrated embodiment, the processor **201,** the non-volatile memory device **202,** the communications device **203,** and the power supply **204** are housed in a housing or a case **205,** and the proximal end(s) **302** of the electrical lead(s) **301** extend through opening(s) **206** in the case **205** and are connected to the power supply 204.

In the illustrated embodiment, the IPG **200** also includes an inertial sensing unit (or inertial sensor) **207** in the case **205** that is configured to determine (e.g., measure or calculate) physiological data, such as one or more physiological signals of the patient, such as respiration rate or heart rate. In one or more embodiments, the inertial sensing unit **207** may be an accelerometer and/or a gyroscope (e.g., the inertial sensing unit **207** may be an inertial measurement unit (IMU) including an accelerometer and a gyroscope). In one or more embodiments, the IPG **200** may include a plurality of inertial sensing units **207** configured to determine (e.g., measure or calculate) a plurality of physiological signals of the patient. In the illustrated embodiment, the IPG **200** also includes a timer **208** (e.g., a real-time clock (RTC)) in the case **205** that is configured to measure the passage of time. The inertial sensing unit **207** and the timer **208** are in communication with the processor 201.

As used herein, the term "processor" includes any combination of hardware, firmware, memory and software, employed to process data or digital signals. The hardware of a controller may include, for example, a microcontroller, application specific integrated circuits (ASICs), general purpose or special purpose central processors (CPUs), digital signal processors (DSPs), graphics processors (GPUs), and programmable logic devices such as field programmable gate arrays (FPGAs). In a processor, as utilized herein, each function is performed either by hardware configured, i.e., hard-wired, to perform that function, or by more general purpose hardware, such as a CPU, configured to execute instructions stored in a non-transitory storage medium or memory. A processor may contain two or more processors, for example, a processor may include two processors, an FPGA and a CPU, interconnected on a PCB.

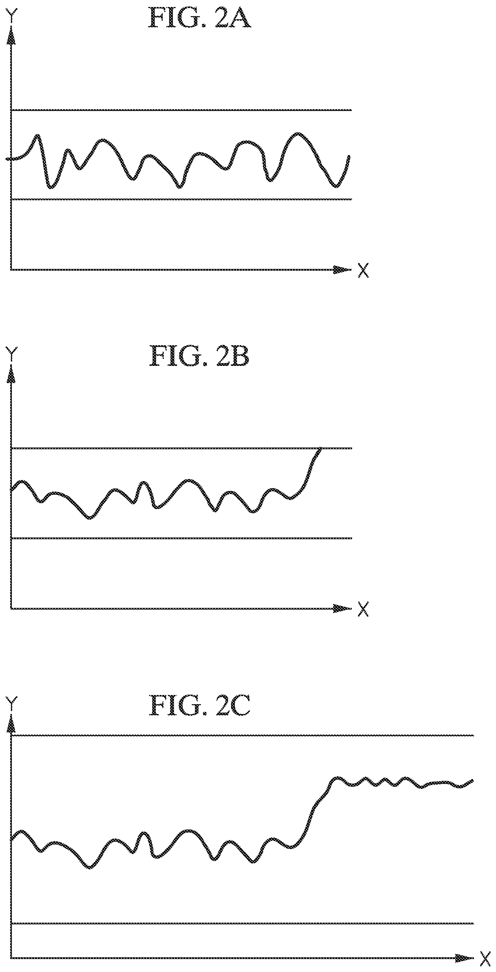

In one or more embodiments, the memory device **202** includes computer-readable instructions (e.g., software code) which, when executed by the processor **201,** cause the IPG **200** to capture (e.g., receive or record) the physiological signal(s) from the inertial sensing unit(s) **207** within an initial range between a maximum value and a minimum value (i.e., receive the physiological signal(s) from the inertial sensing unit(s) **207** within an initial scale). In one or more embodiments, the initial range may be relatively narrow such that the resolution is relatively high (e.g., the range may be a minimum range in which the physiological signals are anticipated to lie). For instance, in one or more embodiments in which the inertial sensing unit **207** is (or includes) an accelerometer, the range may be from -2G to +2G. FIG. 2A depicts the physiological signal, determined by the inertial sensor, within the initial range. Sampling the physiological signals at a relatively narrow range increases the resolution and sensitivity of the IPG **200,** which may enable detection of low-intensity physiological signals, such as physiological signals when the patient is asleep. In one or more embodiments in which the IPG **200** includes two or more inertial sensing unit **207,** the computer-readable instructions, when executed by the processor **201,** cause the IPG **200** to capture the physiological signals through two or more channels (e.g., one channel for each physiological signal).

In one or more embodiments, the memory device **202** includes computer-readable instructions, when executed by the processor **201,** cause the IPG **200** to dynamically increase the range in response to the physiological signal reaching (or approaching) the maximum value or the minimum value of the range. In one or more embodiments in which the IPG **200** includes two or more inertial sensing units **207** that gather physiological signals over two or more channels, the memory device **202** includes computer-readable instructions, when executed by the processor **201,** cause the IPG **200** to dynamically increase the range for the channel(s) that clipped or approached clipping. In one or more embodiments, the instructions, when executed by the processor **201,** cause the IPG **200** to increase the initial range to an enlarged range in response to the physiological signal reaching a threshold maximum value below the maximum value of the range (e.g., approximately 90% to approximately 99% of the maximum value) or reaching a threshold minimum value above the minimum value of the range (e.g., approximately 101% to approximately 110% of the minimum value). In one or more embodiments, the range may be adjustable in fixed increments (e.g., regular increments). For instance, in one or more embodiments in which the inertial sensing unit **207** is (or includes) an accelerometer, the range may increase from the initial range (e.g., -2G to +2G) to an enlarged range (e.g., -4G to +4G). The physiological signal may reach or approach the maximum or minimum value of the range due to bulk movements of the patient, such as when the patient works out or when the patient rolls over while sleeping. Increasing the range is configured to prevent a loss of signal information, which would otherwise occur if the signal reached the maximum or minimum value of the range. That is, increasing the range enables the physiological signal to be recovered. FIG. 2B depicts the physiological signal when it has clipped the first range (e.g., the physiological signal reached the maximum (or the maximum threshold) or the minimum (or the minimum threshold)), and FIG. 2C depicts the physiological signal when the range has been increased.

In one or more embodiments, the memory device **202** includes computer-readable instructions which, when executed by the processor **201,** cause the IPG **200** to initiate a timer, utilizing the timer **208,** in response to the initial range being increased to the enlarged range. That is, in one or more embodiments, the computer-readable instructions, when executed by the processor **201,** cause the IPG **200** to initiate a timer in response to one of the physiological signals clipping (i.e., hitting the maximum or minimum value of the initial range) or approach clipping (i.e., reaching a threshold maximum value below the maximum value of the range or reaching a threshold minimum value above the minimum value of the range). In one or more embodiments, the timer may be 15 seconds or less (e.g., in a range from 5 seconds to 10 seconds). In one or more embodiments, the timer may be set based on maximum observed patient motion data.

In one or more embodiments, the memory device **202** includes computer-readable instructions which, when executed by the processor **201,** cause the IPG **200** to reduce the enlarged range back to the initial range following expiration of the timer (e.g., 15 seconds or less). The timer is configured to account for the expected time that it will take for the bulk movements of the patient, such as rolling over while sleeping, to cease. Accordingly, reducing the range after the timer expires is configured to increase the resolution and sensitivity of the inertial sensor(s) while avoiding (or at least mitigating the risk of) the physiological signal clipping.

In one or more embodiments, the memory device **202** includes computer-readable instructions which, when executed by the processor **201,** cause the IPG **200** to determine that the physiological signal is within a range narrower than the enlarged range. For example, in one or more embodiments in which the range may be adjusted in fixed increments (e.g., regular increments), the computer-readable instructions, when executed by the processor **201,** cause the IPG **200** to determine that the physiological signal is within a range that it is at least one increment lower than the enlarged range. In one or more embodiments, the computer-readable instructions, when executed by the processor **201,** cause the IPG **200** to determine that the physiological signal is within the initial range (e.g., the physiological signal has returned to the initial range after exceeding the initial range due to the bulk movement of the patient). As described above, reducing the range is configured to increase the resolution and sensitivity of the inertial sensing unit(s)) **207,** which may enable detection of low-intensity physiological signals (e.g., physiological signals when the patient is asleep), while avoiding (or at least mitigating the risk of) the physiological signal clipping.

In one or more embodiments, the memory device **202** includes computer-readable instructions which, when executed by the processor **201,** cause the IPG **200** to separate or differentiate the physiological signal from the portion of the signal attributed to the bulk movement of the patient (e.g., due to exercise or movement while sleeping). In one or more embodiments, the computer-readable instructions utilize a bandpass filter to separate the physiological signal from the portion of the signal attributed to the bulk movement of the patient. In one or more embodiments, the IPG **200** may utilize a software-based bypass filter or a physical bandpass filter device to separate the physiological signal from the portion of the signal attributed to the bulk movement of the patient. In one or more embodiments, the bandpass filter may have corner frequencies at 0.1 Hz and 0.5 Hz. In one or more embodiments, the bandpass filter has corner frequencies at 0.2 Hz and 50 Hz. In one or more embodiments, an adaptive filter may be utilized. For instance, in one or more embodiments in which the physiological signal measured by the inertial sensing unit **207** is respiration rate, an adaptive filter may be applied to adjust the corner frequencies based on the rise and fall time of the first 4 seconds (1 breath cycle) of the physiological signal.

In one or more embodiments, the memory device **202** includes computer-readable instructions which, when executed by the processor **201,** cause the IPG **200** to stimulate a nerve and/or a muscle in the patient, via the one or more electrodes **303,** based on the analysis of the one or more physiological signals. For instance, in one or more embodiments in which the IPG **200** is an obstructive sleep apnea (OSA) stimulator device and the inertial sensing unit **207** is configured to measure the respiration rate of the patient, the IPG **200** may apply electrical stimulation, via the one or more electrodes **303,** to the upper airway dilator muscles of the patient in response to the respiration rate signal received by the inertial sensing unit **207** indicating that sleep apnea is occurring. In one or more embodiments in which the IPG **200** is a Vagus nerve stimulation (VNS) device and the inertial sensing unit **207** is configured to measure the heart rate of the patient, the IPG **200** may apply electrical stimulation, via the one or more electrodes **303,** to the patient's Vagus nerve or hypoglossal nerve to shorten or lessen the severity of a possible seizure in response to analysis of the heart rate signal indicating ictal tachycardia is occurring.

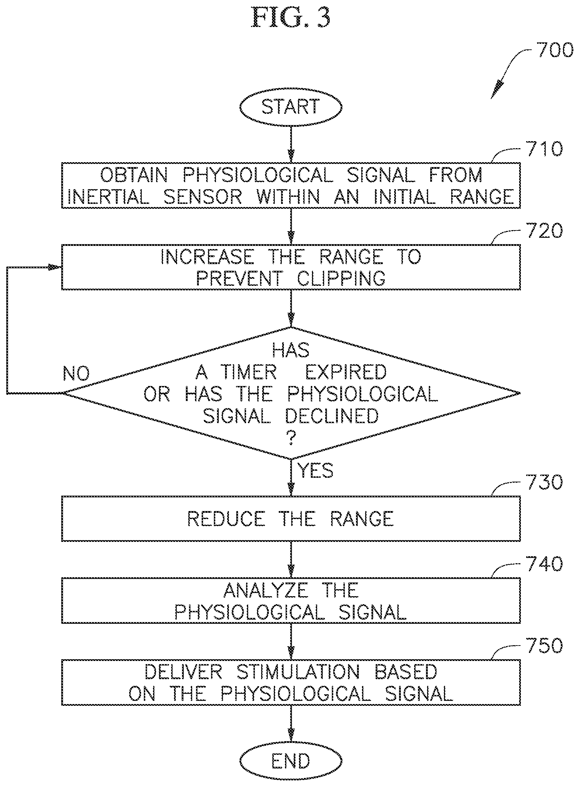

FIG. 3 depicts a flowchart illustrating tasks of a method **700** of operating a medical device implanted in a patient. The medical may be an implantable pulse generator (IPG) implanted in a subcutaneous pocket in the upper chest of the patient. The medical device may be the IPG **200** and the implantable lead system **300** described above with reference to FIGS. 1A-1B.

In the illustrated embodiment, the method **700** includes a task **710** of obtaining (e.g., receiving or recording) physiological data, such as at least one physiological signal or indicator, from at least one inertial sensing unit (or inertial sensor) of the implantable medical device. The physiological signal obtained in task **710** may be, for example, a respiration signal or a heart rate signal. The physiological signal obtained in task **710** is within an initial range (i.e., an initial range between an initial minimum and an initial maximum). In one or more embodiments, the initial range may be a relatively narrow range (e.g., a minimum range) that is intended to encompass the fully physiological signal during normal operation. As described above, the relatively narrow initial range increases (e.g., maximizes) the resolution and thus the sensitivity of the implantable device, which may enable detection of low-intensity physiological signals, such as physiological signals when the patient is asleep.

In one or more embodiments, the method **700** also includes a task **720** of increasing the range from the initial range to an enlarged range (e.g., a maximum that is greater than the initial maximum and a minimum that is lower than the initial minimum) in response to the physiological signal reaching the maximum value or the minimum value of the range. In this manner, the task **720** is configured to prevent the physiological signal from clipping, which would otherwise result in the loss of signal information. For instance, the task **720** of increasing the range may be in response to the physiological signal reaching the maximum value of the initial range (or reaching a threshold maximum value below the maximum value of the range, such as approximately 90% to approximately 99% of the maximum value) or reaching the minimum value of the initial range (or reaching a threshold minimum value above the minimum value of the range, such as approximately 101% to approximately 110% of the minimum value). As described above, increasing the range is configured to prevent a loss of signal information, which would otherwise occur if the signal reached the maximum or minimum value of the range. In one or more embodiments, the task **720** may include increasing the range in fixed increments (e.g., regular increments). For instance, in one or more embodiments in which the inertial sensing unit is (or includes) an accelerometer, the task may include increasing from an initial range of -2G to +2G to a range from -4G to +4G.

In one or more embodiments, the method **700** also includes a task **730** of reducing the range (e.g., reducing the enlarged range back to the initial range). The task **730** of reducing the range may be performed in response to a timer expiring. The timer may have been initiated immediately after the task of increasing the range or simultaneously with the task of increasing the range. In one or more embodiments, the timer may be in a range from approximately 5 seconds to approximately 15 seconds. In one or more embodiments, the task **730** of reducing the range may be performed in response to the physiological signal dropping from within the enlarged range to a narrower range. In one or more embodiments, the task **730** of reducing the range may be performed in response to both the timer expiring and the signal analysis indicating that the physiological signal has dropped from within the enlarged range to within a narrower range. The physiological signal may drop once the bulk movement of the patient, such as exercising or rolling over while sleeping, has ceased.

In one or more embodiments, the method **700** also includes a task **740** of analyzing the physiological signal. In one or more embodiments, the task **740** of analyzing the physiological signal includes separating or differentiating the physiological signal from the portion of the signal attributed to the bulk movement of the patient (e.g., due to exercise or movement while sleeping). In one or more embodiments, the task **740** includes utilizing a bandpass filter (e.g., a software-based bandpass filter or a physical bandpass filter device) to separate the physiological signal from the portion of the signal attributed to the bulk movement of the patient.

In one or more embodiments, the method **700** also includes a task **750** of stimulating the patient based on the analysis of the physiological signal in task 740. The task **750** may include applying electrical stimulation, via the one or more electrodes, to the upper airway dilator muscles of the patient in response to the respiration rate signal received by the inertial sensing unit indicating that sleep apnea is occurring. In one or more embodiments, the task **750** may include applying electrical stimulation, via the one or more electrodes, to the patient's Vagus nerve to shorten or lessen the severity of a possible seizure in response to analysis of the heart rate signal indicating ictal tachycardia is occurring.

The implantable pulse generator and/or any other relevant devices or components according to embodiments of the present invention described herein may be implemented utilizing any suitable hardware, firmware (e.g., an application-specific integrated circuit), software, or a combination of software, firmware, and hardware. For example, the various components of the device may be formed on one integrated circuit (IC) chip or on separate IC chips. Further, the various components of the device may be implemented on a flexible printed circuit film, a tape carrier package (TCP), a printed circuit board (PCB), or formed on one substrate. Further, the various components of the device may be a process or thread, running on one or more processors, in one or more computing devices, executing computer program instructions and interacting with other system components for performing the various functionalities described herein. The computer program instructions are stored in a memory which may be implemented in a computing device using a standard memory device, such as, for example, a random access memory (RAM). The computer program instructions may also be stored in other non-transitory computer readable media such as, for example, a CD-ROM, flash drive, or the like.

Although some embodiments of the present disclosure are disclosed herein, the present disclosure is not limited thereto, and the scope of the present disclosure is defined by the appended claims.

## Claims

1. A medical device comprising:
an implantable pulse generator comprising:
a processor:
a non-volatile memory device coupled to the processor;
a power supply coupled to the processor; and
an inertial sensing unit coupled to the processor, the inertial sensing unit being configured to measure one or more physiological indicators of a patient; and
an implantable lead system comprising:
at least one electrical lead coupled to the power supply of the implantable pulse generator; and
at least one electrode at a distal end of the at least one electrical lead,
wherein the non-volatile memory device comprises instructions stored therein which, when executed by the processor, cause the implantable pulse generator to:
receive the one or more physiological indicators within an initial range of values of the one or more physiological indicators from the inertial sensing unit;
increase the initial range to an enlarged range of values of the one or more physiological indicators in response to at least one of the one or more physiological indicators crossing a threshold maximum value or a threshold minimum value of the initial range;
receive the one or more physiological indicators within the enlarged range,
analyze the one or more physiological indicators, and
deliver stimulation to the patient through the at least one electrode in response to the one or more physiological indicators.

2. The implantable pulse generator of claim 1, wherein the instructions, when executed by the processor, further cause the implantable pulse generator to distinguish the one or more physiological indicators from broad-spectrum noise in the enlarged range utilizing a bandpass filter.

3. The implantable pulse generator of claim 2, wherein the bandpass filter has corner frequencies at 0.1 Hz and 0.5 Hz.

4. The implantable pulse generator of claim 2, wherein the bandpass filter has corner frequencies at 0.2 Hz and 50 Hz.

5. The implantable pulse generator of any one of claim 2, claim 3, or claim 4, wherein the bandpass filter is an adaptive bandpass filter.

6. The implantable pulse generator of any one of the preceding claims, wherein the at least one inertial sensing unit comprises an accelerometer.

7. The implantable pulse generator of claim 6, wherein the initial range is -2G to +2G.

8. The implantable pulse generator of claim 7, wherein the enlarged range is -4G to +4G.

9. The implantable pulse generator of any one of the preceding claims, wherein the at least one inertial sensing unit comprises a gyroscope.

10. The implantable pulse generator of any one of the preceding claims, wherein the one or more physiological indicators comprise respiration rate, and wherein the instructions, when executed by the processor, cause the implantable pulse generator to deliver electrical stimulation through the at least one implantable lead to upper airway dilator muscles of the patient.

11. The implantable pulse generator of any one of the preceding claims, wherein the one or more physiological indicators comprise heart rate, and wherein the instructions, when executed by the processor, cause the implantable pulse generator to deliver electrical stimulation through the at least one implantable lead to the patient's Vagus nerve or hypoglossal nerve.

12. The implantable pulse generator of claim 1, wherein the at least one inertial sensing unit comprises a plurality of inertial sensing units configured to measure a plurality of the physiological indicators of the patient.

13. The implantable pulse generator of claim 1, wherein the instructions, when executed by the processor, further cause the implantable pulse generator to:
initiate a timer in response to initial range being increased to the enlarged range; and
decrease the enlarged range to the initial range in response to the timer expiring.

14. The implantable pulse generator of claim 1, wherein the instructions, when executed by the processor, further cause the implantable pulse generator to decrease the initial range to a narrower range less than the initial range in response to at least one of the one or more physiological indicators being within the narrower range.

15. The implantable pulse generator of claim 1, wherein the instructions, when executed by the processor, cause the implantable pulse generator to adjust the initial range in regular increments.

## Patentansprüche

1. Medizinische Vorrichtung, umfassend:
einen implantierbaren Impulsgenerator, umfassend:
einen Prozessor;
eine nichtflüchtige Speichervorrichtung, die mit dem Prozessor gekoppelt ist;
eine Leistungsversorgung, die mit dem Prozessor gekoppelt ist; und
eine inertiale Abfühleinheit, die mit dem Prozessor gekoppelt ist, wobei die inertiale Abfühleinheit dazu ausgelegt ist, einen oder mehrere physiologische Indikatoren eines Patienten zu messen; und
ein implantierbares Zuleitungssystem, umfassend:
zumindest eine elektrische Zuleitung, die mit der Leistungsversorgung des implantierbaren Impulsgenerators gekoppelt ist; und
zumindest eine Elektrode an einem distalen Ende der zumindest einen elektrischen Zuleitung, wobei die nichtflüchtige Speichervorrichtung darauf gespeicherte Befehle umfasst, die, wenn sie durch den Prozessor ausgeführt werden, bewirken, dass der implantierbare Impulsgenerator:
den einen oder die mehreren physiologischen Indikatoren innerhalb eines anfänglichen Wertebereichs des einen oder der mehreren physiologischen Indikatoren von der inertialen Abfühleinheit empfängt;
den anfänglichen Bereich als Reaktion auf das Überschreiten eines Schwellenhöchstwerts oder das Unterschreiten eines Schwellenmindestwerts des anfänglichen Bereichs durch den einen oder die mehreren Indikatoren auf einen vergrößerten Wertebereich des einen oder der mehreren physiologischen Indikatoren erhöht;
den einen oder die mehreren physiologischen Indikatoren innerhalb des vergrößerten Bereichs empfängt,
den einen oder die mehreren physiologischen Indikatoren analysiert und
als Reaktion auf den einen oder die mehreren physiologischen Indikatoren über die zumindest eine Elektrode eine Stimulierung an den Patienten abgibt.

2. Implantierbarer Impulsgenerator nach Anspruch 1, wobei die Befehle, wenn sie durch den Prozessor ausgeführt werden, ferner bewirken, dass der implantierbare Impulsgenerator unter Verwendung eines Bandpassfilters den einen oder die mehreren physiologischen Indikatoren von Breitspektrumrauschen in dem vergrößerten Bereich unterscheidet.

3. Implantierbarer Impulsgenerator nach Anspruch 2, wobei der Bandpassfilter Eckfrequenzen bei 0,1 Hz und 0,5 Hz aufweist.

4. Implantierbarer Impulsgenerator nach Anspruch 2, wobei der Bandpassfilter Eckfrequenzen bei 0,2 Hz und 50 Hz aufweist.

5. Implantierbarer Impulsgenerator nach einem von Anspruch 2, Anspruch 3 oder Anspruch 4, wobei der Bandpassfilter ein adaptiver Bandpassfilter ist.

6. Implantierbarer Impulsgenerator nach einem der vorangegangenen Ansprüche, wobei die zumindest eine inertiale Abfühleinheit einen Beschleunigungsmesser umfasst.

7. Implantierbarer Impulsgenerator nach Anspruch 6, wobei der anfängliche Bereich -2G bis +2G ist.

8. Implantierbarer Impulsgenerator nach Anspruch 7, wobei der vergrößerte Bereich -4G bis +4G ist.

9. Implantierbarer Impulsgenerator nach einem der vorangegangenen Ansprüche, wobei die zumindest eine inertiale Abfühleinheit ein Gyroskop umfasst.

10. Implantierbarer Impulsgenerator nach einem der vorangegangenen Ansprüche, wobei der eine oder die mehreren physiologischen Indikatoren die Atmungsfrequenz umfassen und wobei die Befehle, wenn sie durch den Prozessor ausgeführt werden, bewirken, dass der implantierbare Impulsgenerator über die zumindest eine implantierbare Zuleitung eine elektrische Stimulierung an die erweiternden Muskeln der oberen Atemwege des Patienten abgibt.

11. Implantierbarer Impulsgenerator nach einem der vorangegangenen Ansprüche, wobei der eine oder die mehreren physiologischen Indikatoren die Herzfrequenz umfassen und wobei die Befehle, wenn sie durch den Prozessor ausgeführt werden, bewirken, dass der implantierbare Impulsgenerator durch die zumindest eine implantierbare Zuleitung eine elektrische Stimulierung an den Vagusnerv oder den Unterzungennerv des Patienten abgibt.

12. Implantierbarer Impulsgenerator nach Anspruch 1, wobei die zumindest eine inertiale Abfühleinheit eine Vielzahl von inertialen Abfühleinheiten umfasst, die dazu ausgelegt sind, eine Vielzahl der physiologischen Indikatoren des Patienten zu messen.

13. Implantierbarer Impulsgenerator nach Anspruch 1, wobei die Befehle, wenn sie durch den Prozessor ausgeführt werden, ferner bewirken, dass der implantierbare Impulsgenerator:
als Reaktion auf das Vergrößern des anfänglichen Bereichs auf den vergrößerten Bereich eine Zeitgebung initiiert; und
als Reaktion auf das Ablaufen der Zeitgebung den vergrößerten Bereich auf den anfänglichen Bereich verkleinert.

14. Implantierbarer Impulsgenerator nach Anspruch 1, wobei die Befehle, wenn sie durch den Prozessor ausgeführt werden, ferner bewirken, dass der implantierbare Impulsgenerator den anfänglichen Bereich auf einen kleineren Bereich als den anfänglichen Bereich verkleinert, als Reaktion darauf, dass zumindest einer aus dem einen oder den mehreren physiologischen Indikatoren innerhalb des kleineren Bereichs liegt.

15. Implantierbarer Impulsgenerator nach Anspruch 1, wobei die Befehle, wenn sie durch den Prozessor ausgeführt werden, bewirken, dass der implantierbare Impulsgenerator den anfänglichen Bereich in regelmäßigen Inkrementen anpasst.

## Revendications

1. Dispositif médical, comprenant :
un générateur d'impulsions implantable, comprenant :
un processeur ;
un dispositif de mémoire non volatile couplé au processeur ;
une alimentation électrique couplée au processeur ; et
une unité de détection inertielle couplée au processeur, l'unité de détection inertielle étant configurée pour mesurer un ou plusieurs indicateurs physiologiques d'un patient ; et
un système de sonde implantable comprenant :
au moins une sonde électrique couplé à l'alimentation électrique du générateur d'impulsions implantable ; et
au moins une électrode au niveau d'une extrémité distale de la au moins une sonde électrique,
dans lequel le dispositif de mémoire non volatile comprend des instructions stockées dans celui-ci qui, lorsqu'elles sont exécutées par le processeur, amènent le générateur d'impulsions implantable à :
recevoir les un ou plusieurs indicateurs physiologiques dans une plage initiale de valeurs des un ou plusieurs indicateurs physiologiques depuis l'unité de détection inertielle ;
augmenter la plage initiale à une plage élargie de valeurs des un ou plusieurs indicateurs physiologiques en réponse au franchissement par au moins un des un ou plusieurs indicateurs physiologiques d'une valeur maximale de seuil ou d'une valeur minimale de seuil de la plage initiale ;
recevoir les un ou plusieurs indicateurs physiologiques dans la plage élargie,
analyser les un ou plusieurs indicateurs physiologiques, et
administrer une stimulation au patient par l'intermédiaire de la au moins une électrode en réponse aux un ou plusieurs indicateurs physiologiques.

2. Générateur d'impulsions implantable selon la revendication 1, dans lequel les instructions, lorsqu'elles sont exécutées par le processeur, amènent en outre le générateur d'impulsions implantable à distinguer les un ou plusieurs indicateurs physiologiques d'un bruit à large spectre dans la plage élargie en utilisant un filtre passe-bande.

3. Générateur d'impulsions implantable selon la revendication 2, dans lequel le filtre passe-bande présente des fréquences de coupure à 0,1 Hz et 0,5 Hz.

4. Générateur d'impulsions implantable selon la revendication 2, dans lequel le filtre passe-bande présente des fréquences de coupure à 0,2 Hz et 50 Hz.

5. Générateur d'impulsions implantable selon l'une quelconque des revendications 2, 3 ou 4, dans lequel le filtre passe-bande est un filtre passe-bande adaptatif.

6. Générateur d'impulsions implantable selon l'une quelconque des revendications précédentes, dans lequel la au moins une unité de détection inertielle comprend un accéléromètre.

7. Générateur d'impulsions implantable selon la revendication 6, dans lequel la plage initiale est de -2G à +2G.

8. Générateur d'impulsions implantable selon la revendication 7, dans lequel la plage élargie est de -4G à +4G.

9. Générateur d'impulsions implantable selon l'une quelconque des revendications précédentes, dans lequel la au moins une unité de détection inertielle comprend un gyroscope.

10. Générateur d'impulsions implantable selon l'une quelconque des revendications précédentes, dans lequel les un ou plusieurs indicateurs physiologiques comprennent une fréquence de respiration, et dans lequel les instructions, lorsqu'elles sont exécutées par le processeur, amènent le générateur d'impulsions implantable à administrer une stimulation électrique à travers la au moins une sonde implantable aux muscles dilatateurs des voies respiratoires supérieures du patient.

11. Générateur d'impulsions implantable selon l'une quelconque des revendications précédentes, dans lequel les un ou plusieurs indicateurs physiologiques comprennent la fréquence cardiaque, et dans lequel les instructions, lorsqu'elles sont exécutées par le processeur, amènent le générateur d'impulsions implantable à administrer une stimulation électrique à travers la au moins une sonde implantable au nerf vague ou au nerf hypoglosse du patient.

12. Générateur d'impulsions implantable selon la revendication 1, dans lequel la au moins une unité de détection inertielle comprend une pluralité d'unités de détection inertielles configurées pour mesurer une pluralité d'indicateurs physiologiques du patient.

13. Générateur d'impulsions implantable selon la revendication 1, dans lequel les instructions, lorsqu'elles sont exécutées par le processeur, amènent en outre le générateur d'impulsions implantable à :
initier une minuterie en réponse à l'augmentation de la plage initiale jusqu'à la plage élargie ; et
réduire la plage élargie jusqu'à la plage initiale en réponse à l'expiration de la minuterie.

14. Générateur d'impulsions implantable selon la revendication 1, dans lequel les instructions, lorsqu'elles sont exécutées par le processeur, amènent en outre le générateur d'impulsions implantable à réduire la plage initiale à une plage plus étroite inférieure à la plage initiale en réponse à au moins un des un ou plusieurs indicateurs physiologiques étant dans la plage plus étroite.

15. Générateur d'impulsions implantable selon la revendication 1, dans lequel les instructions, lorsqu'elles sont exécutées par le processeur, amènent le générateur d'impulsions implantable à ajuster la plage initiale par incréments réguliers.
